# EUROPÄISCHE PATENTSCHRIFT

(11) **EP 2 836 196 B1**
(45) Veröffentlichungstag und Bekanntmachung des Hinweises auf die Patenterteilung: **12.06.2019**
(21) Anmeldenummer: 13714213.9
(22) Anmeldetag: 03.04.2013
(51) Int. Cl.: A61K 9/00, A61K 31/785, A61P 31/02

(54) **ZUBEREITUNG ZUR TOPISCHEN ANWENDUNG AUF SCHLEIMHÄUTEN MIT POLYHEXANID ALS WIRKSTOFF**
PREPARATION FOR TOPICAL APPLICATION TO MUCOSA CONTAINING POLYHEXANIDE AS ACTIVE INGREDIENT
PRÉPARATION POUR APPLICATION LOCALE SUR LES MUQUEUSES, CONTENANT UN POLYHEXANIDE COMME SUBSTANCE ACTIVE

(30) Priorität: 11.04.2012 DE 102012007212
(43) Veröffentlichungstag der Anmeldung: 18.02.2015
(73) Patentinhaber: Merz Pharma GmbH & Co. KGaA, 60318 Frankfurt (DE)
(72) Erfinder: FUNKE, Bettina, 60316 Frankfurt (DE)
(74) Vertreter: Müller, Claudia
(86) Internationale Anmeldenummer: PCT/EP2013/000986
(87) Internationale Veröffentlichungsnummer: WO 2013/152838

(56) Entgegenhaltungen:
- EP-A1- 0 788 797
- EP-A2- 0 181 179
- WO-A1-94/27440
- DE-A1- 10 012 026
- DE-A1-102004 037 598
- DE-A1-102004 052 308
- DE-U1-202009 000 862
- JP-A- 2004 026 725

## Beschreibung

### Gegenstand der Erfindung

Die vorliegende Erfindung betrifft eine bakteriophagenfreie Zubereitung zur topisch-oralen Anwendung im nicht gefrorenen Zustand auf Schleimhäuten im Mund-Nasen- und Rachenraum bei Menschen,dadurch gekennzeichnet, dass sie Polyhexanid (Polyhexamethylenbiguanid), oder ein wasserlösliches Salz hiervon, als antiinfektiven Wirkstoff, mit einer molaren Masse von 2300 bis 3100 g/mol und einen oder mehrere Zusatzstoffe, ausgewählt aus Aroma-/Geschmacksstoffen, Vitaminen, sowie galenischen Hilfsstoffen, ausgewählt aus Zuckern, Zuckeraustauschstoffen, Zuckerersatzstoffen, Säuerungsmitteln, Lösungsvermittlern, Verdickern, Süßungsmitteln, Farbstoffen, Konservierungsmitteln, oder Kombinationen hiervon aufweist, wobei die Zubereitung im Wesentlichen frei ist von weiteren antiinfektiven Wirkstoffen, und als feste Dareichungsform mit Zuckern und/oder Zuckeraustauschstoffen als Träger vorliegt. Besondere stabilisierende galenische Zusatzstoffe wie bestimmte lipidische Komponenten wie z. B. Phospholipide sind hierbei nicht erforderlich. Das Mittel kann Polyhexanid in Mengen aufweisen, welche eine gute Wirksamkeit, jedoch keine toxischen Effekte bedingen. Geeignet sind die Zubereitungen bei Reizungen wie Halsschmerzen, die eventuell auch mit Heiserkeit, Halskratzen einhergehen, die durch potenziell pathogene Mikroorganismen gesunder Erwachsener in Mund oder Rachen (Bakterien wie z.B. alphahämolysierende Streptokokken, Streptococcus pyogenes, Streptococcus pneumoniae, Pseudomonas aeruginosa, Staphylococcus aureus, Candida albicans, Enterococcus faecalis, Haemophilus influenza, Candida albicans, Staphylococcus aureus, nicht jedoch Chlamydien), oder auch bei Reizzuständen, die durch die auf die Infektion folgende Entzündung verursacht werden.

### Stand der Technik

Als Wirkstoffe zur topischen Behandlung von entzündlichen Prozessen des Mund- und Rachenraums ist vor allem Cetylpyridiniumchlorid (1-Hexadecylpyridinium-chlorid), z.B. in Form von Lutschtabletten bekannt. Dieser Wirkstoff kann jedoch Magen-Darmbeschwerden auslösen. Ein anderer zum genannten Zweck bekannter Wirkstoff ist Hexidin (1,3-Bis(2-ethyl-hexyl)-hexahydro-5-methyl-5-pyridin-amin), z. B. in Form eines Sprays oder als Gurgellösung. Hexidin kann jedoch zu Geschmacksirritationen führen (siehe auch EP 1799186, Einleitung).

Darüberhinaus werden auch Wirkstoffe natürlichen Ursprungs eingesetzt wie Pflanzenextrakte, z. B. Isländisch Moos, Salbei u.ä.. Dabei werden aber nicht immer die gewünschten Erfolge erzielt.

Ein weiterer Wirkstoff mit antiinfektivem Potential ist Polyhexanid (Polyhexamethylenbiguanid). Dieser ist als desinfizierender Wirkstoff für Oberflächen, und vor allem auch als Wundantiseptikum oder Mittel für die Herstellung von Wundauflagen bekannt. Dabei wird Polyhexanid meist in wässriger Lösung, z.B. als Hydrochlorid, zusammen mit Zusatzstoffen wie Polyethylenglykol eingesetzt, siehe auch EP B 0700249 B1, betreffend ein Antiinfektivum, insbesondere ein Wundbehandlungsmittel oder ein iv-einzusetzendes Mittel, enthaltend Polyhexanid (Polyhexamethylenbiguanid) mit einer mittleren Molmasse von 2900 bis 15000, vor allem auch in Kombination mit weiteren Wirkstoffen.

Die DE 10 2009 005 862 A1 betrifft Einheiten mit gefrorenen festen wässrigen Dosierungsformen, welche sich bei der Anwendung im Rachenraum durch die dortige Wärme auflösen und daher als Lutschbonbon eingesetzt werden können. Dabei können vor allem kühlende Wirkstoffe wie ätherische Öle (Menthol etc.), Pflanzenstoffe oder auch chemische Antiinfektiva wie z.B. Polyhexanid, insbesondere Kombinationen derartiger Wirkstoffe vorhanden sein.

Die DE 10 2009 008 919 A1 betrifft wässrige Mittel mit einem pH-Wert von 2,5 bis 3,5, ent-haltend Eisen, Zink (Verbindungen), eine Säure, ein Tensid sowie Polyhexanid zur Behandlung chronischer oder akuter Wunden. Aus der DE 10 2008 064 481A1 sind Mittel bekannt, welche zwingend Vitamine, mindestens eine Metallverbindung (aus der Gruppe IV, Nebengruppe I, II, VIII des Periodensystems wie Kobalt, Eisen, Kupfer, Silber) sowie ein Tensid und ein Desinfektionsmittel wie ein Biguanid aufweisen können. Derartige Kombinationsmittel sollen als Desinfektionsmittel für unbelebte und belebte Oberflächen (wie Haut, Haare, Schleimhäute) geeignet sein.

Die DE 10 2008 005 193 A1 offenbart Zubereitungen aus lytischen Bakteriophagen und Polyhexanid, welche topisch im Mund-Rachenraum auf Haut/Schleimhaut angewendet werden können. Dabei muss die Polyhexanid-Konzentration so gering sein, dass sie die externen Bakterien hemmt, aber die internen Bakteriophagen nicht zerstört werden.

Die Veröffentlichungen DE 10 2006 015 271 A1 bzw. EP 2 001 440 B1 beschreiben Polyhexanid- beladene Liposomen, insbesondere zum Einsatz bei der Wundbehandlung. Für Polyhexanid wird hier festgestellt, dass dessen Wirkung eher verlangsamt eintritt.

Aus der DE 296 112 66 U1 sind Polyhexanid- haltige Mittel zur Wundbehandlung bekannt, deren Träger eine höhere Viskosität als Wasser aufweisen, wie z.B. Cremes oder Salben. Damit soll eine bessere Hautverträglichkeit erzielt werden. Das Europäische Patent EP 1214055 B1 schützt Phospholipid- und Vitaminhaltige Polyhexanid-Gele, die eine verbesserte Hautverträglichkeit aufweisen.

Die DE 19936545 A1 betrifft im Wesentlichen wasserfreie Polyhexanid- Zubereitungen zur nasalen Anwendung, welche keine kürzeren Alkohole, jedoch Neutralöle (Trigylceride) aufweisen.

Das Europäische Patent EP 1 799 186 B1 (Priorität: DE 10 2004052308 A1) betrifft Lutschtabletten zur Anwendung bei Hals- und Rachenerkrankungen mit Octenidin als Wirkstoff. Octenidin kann jedoch auch zyto- und gewebetoxische Nebenwirkungen, vergleichbar mit Chlorhexidin, mit sich bringen, siehe Kramer A., Adrian V., Rudolph P., Wurster S., Lippert H., Explantationstest mit Haut und Peritoneum der neonatalen Ratte als Voraussagetest zur Verträglichkeit lokaler Antiinfektiva für Wunden und Körperhöhlen, Chirurg. 1998b; 69:840-5', insbesondere Tab. 49.2.

Aus der EP 0 788 797 A1 ist die Verwendung von Polyhexanid (z. B in Form von Tropfen, Gelen, Sprühlösungen, Emulsionen, Cremes, ggf. mit Polyethylenglykol) mit einem Molekulargewicht von maximal 5000 bei Infektionen durch sich intrazellular vermehrende Bakterien (wie Chlamydien), im Auge, Urogenitaltrakt, Peritonealtrakt bekannt.

Die EP 1575600 B1 betrifft kosmetische Wirkstoffkombinationen aus Polyhexamethylenbiguanid-Hydro-Chlorid und Distearyldimethylammoniumchlorid in Form von O/W-Emulsionen als antibakterielle, antimycotische oder antivirale Wirkstoffe.

Weiterhin bekannt ist auf dem deutschsprachigen Markt die Anwendung von Polyhexanid in Kombination mit mindestens einem weiteren Wirkstoff wie quaternäre Ammoniumsalze sowie einem Tensid in der Mundhöhle. Dort erreicht es z.T. die Wirksamkeit von Chlorhexidin bei gleichzeitiger Plaquehemmung, speziell in der Langzeitanwendung. Rezepturmäßig werden in Apotheken derzeit derartige Polyhexanid-/andere Wirkstoffe enthaltende Augen- und Nasentropfen hergestellt.

Die DE 100 12 026 A1 betrifft ein medizinisches Wasch- oder Duschgel aus 0,01-0,3 Gew. % Polyhexanid, 1 bis 15 % Glycerin, 0,2 bis 5 Gew. % Hydroxyethylcellulose und Wasser.

Aus der DE 10 2004 037 598 A1 sind Mundspüllösungen bekannt, welche in wässriger Lösung Polyhexanid (1500 bis 15000 g/mol) und einen künstlichen Süßstoff wie Acesulfam, Aspartam, Cyclamat zur Verhinderung des Wirkungsverlusts durch den bitteren Wirkstoff-Geschmack maskierender Zusätze aufweisen. Die WO 97/00076 A1 beschreibt die Verwendung von Polyhexanid zur Augenbehandlung, insbesondere als Lösung, Suspension oder Creme.

Rosin, M. et al beschreiben in "The effect of polyhexamethylene biguanide mouthrinse compared to an essential oil rinse and chlorhexidine on bacterial counts and 4-day plaque regrowth" in J. Clin.periodont., 2002, Vol. 98, S. 392-399 eine flüssige Zusammensetzung aus 0,12% Polyhexanid, 0,1% Aromaöl, 0,1 % Emulgatortensid, 10,4% Ethanol als weiteren wirksamen Inhaltsstoff wässriger isotonischer Lösung zur Reduktion von Plaque im Mund-/Zahnbereich. Dabei soll es sich um das Produkt 'Lavasept®' handeln. Dieses umfasst gemäß Müller, G. et al. "Vergleich der bakerioziden Wirksamkeit und In-vitro-Zytotoxizität von Lavasept® und Prontosan®, GMS Krankenhaushygiene Interdisziplinär, 2007, Vol. 2, S. 1-11, Polyhexanid mit einer mittleren Molmasse von 2800 g/Mol und Tensidwirkstoffe (Wirkverstärker).Die US 2004/0009144 A1 betrifft eine pharmazeutische Zubereitung mit einem Polybiguanid-Cyanoguanidin- oder Aminderivat zur Virus- vor allem HIV-Inhibierung.

Aus der DE 39 00 896 A1 ist ein Mundpflegemittel mit einem Polyakylenoxid-Polymertensid und einem Antiinfektivum bekannt. Polyhexanid ist nicht erwähnt.

Die JP 2004/026725 A, insbesondere Abstract, betrifft eine Lutschtablette mit einem kationischen Bakterizid und einem Zuckeralkohol als Träger. Polyhexanid ist nicht genannt.

EP 0181 179 A2 betrifft Mittel zur Behandlung von Gingivitis oder Plaque und offenbart Zubereitungen mit einem Pflanzenöl oder PEG (lubricant), Zucker und Chlorhexidin.

### Aufgabe der Erfindung

Aufgabe vorliegender Erfindung war es, ein Mittel bereitzustellen, welches bei durch Toxine, Staub, Allergene, bakterielle /virale Erreger von Erkältungen und Folgeerscheinungen hiervon u.ä hervorgerufenen Störungen/Entzündungen im Mund- Hals- Nasen- und Rachenraum ohne die bisherigen, durch Wirkstoffe bedingten Nebenwirkungen wie geschildert, insbesondere die durch Octenidin hervorgerufene Gewebebeeinträchtigung des Anwenders zuverlässig eingesetzt werden kann. Ferner sollen aufwendige Herstellungsverfahren wie Liposomen oder besondere galenische Zusätze bzw. Anwendungsformen wie z. B. Lipide, gefrorene Darreichungen vermieden werden. Auch sollen möglichst Kombinationen verschiedener als Antiinfektiva bekannter Wirkstoffe vermieden werden.

### Lösung der Aufgabe

Diese Aufgabe wird erfindungsgemäß dadurch gelöst, dass man Polyhexanid oder insbesondere ein wasserlösliches Salz hiervon, mit einer mittleren molaren Masse von von 2300 bis 3100 g/mol als (insbesondere antiinfektiven) Wirkstoff vor allem in pharmazeutischen Mitteln zum topischen Einsatz auf Schleimhäuten des Menschen, im Mund-/Hals-, Nasen-und/oder Rachenraum einsetzt. Insofern ist das anmeldungsgemäße Mittel/die Zubereitung vor allem im Wesentlichen frei von weiteren antiinfektiven Wirkstoffen wie insbesondere Zink-und/oder Eisenverbindungen (vor allem Salze hiervon) oder Ethanol, Octenidin, Chlorhexidin, oder ähnlichen bekannten antiinfektiven Wirkstoffen. Vorzugsweise ist das Mittel weiterhin im Wesentlichen Lipid- (vor allem Triglycerid)-frei und weist einen pH-Wert von mehr als 3,5, insbesondere 3,7 bis 8, vorzugsweise von 4,3 bis 7,5, insbesondere 4,5 bis 7 (10%ige wässrige Lösung, RT) auf.

Gemäß vorliegender Erfindung bedeutet "pharmazeutisches Mittel" jede Art von pharmazeutischer Zubereitung, vor allem Medizinprodukte, aber auch Arzneimittel, oder auch Kosmetikum, sofern ausdrücklich jeweils benannt.

### Nähere Erläuterung der Erfindung

Die anmeldungsgemäßen Zubereitungen umfassen als Wirkstoff Polyhexanid sowie weiterhin Zusatzstoffe, in Abhängigkeit von der galenischen Darreichungsform. Dabei handelt es sich im Wesentlichen um weitgehend wasserfreie feste Zubereitungen.

Die beschriebenen Zubereitungen liegen demnach im nicht gefrorenen Zustand vor. Sie müssen daher vor der Anwendung auch nicht gekühlt werden. Des Weiteren sind bevorzugt keine Lipide, vorzugsweise ausgewählt aus Trigylceriden, oder auch aus Phospholipiden enthalten. Der Wirkstoff ist vor allem nicht liposomal verpackt.

Die Zubereitungen eignen sich insbesondere zur topisch-oralen unterstützenden/kurativen Anwendung auf Schleimhäuten im Mund- Nasen- Hals und/oder Rachenraum, vor allem bei Reizzuständen, ggf. Entzündungszuständen wie Halsschmerzen. Die Zubereitungen werden zu nicht therapeutischen Zwecken als Kosmetikum oder vor allem therapeutisch, insbesondere als Medizinprodukte eingesetzt. Je nach Einsatzgebiet (Mund-Nase-Rachen) können verschiedene galenische Formen vorliegen wie feste, vorzugsweise lutschbare Formen wie Lutschbonbons, Lutschdragees, insbesondere Karamellen (Mund/ Hals /Rachen). Dazu werden, sofern erforderlich, geeignete Zusatzstoffe inkorporiert. Hierzu gehören Aromen/ Geschmacksstoffe, insbesondere ätherische Öle, Vitamine, sowie galenische Hilfsstoffe, ausgewählt aus Zuckern, Zuckeraustauschstoffen, Zuckerersatzstoffen, Säuerungsmitteln, Lösungsvermittlern wie Wasser, Glykol, Glycerin, Verdickern, Süßungsmitteln, Färbemitteln (Farbstoffen) oder Konservierungsmitteln oder Kombinationen hiervon.

Die Zubereitungen sind insofern vor allem auch im Wesentlichen frei von weiteren antiinfektiven Wirkstoffen.

Überraschenderweise können Reizzustände wie oben erläutert behandelt werden, wenn als Wirkstoff Polyhexanid, oder vorzugsweise ein Salz hiervon, mit einer mittleren molaren Masse wie beschrieben ausgewählt wird. Während der Stand der Technik entweder keine Molmasse angibt oder zum Teil andere, wenn ein direktes Auftragen auf belebte Oberflächen erfolgen soll, war es überraschend, dass mit anmeldungsgemäßen Mitteln überhaupt ein Effekt erzielt werden kann, da diese bzw. Lösungen hiervon zum einen anders als im Stand der Technik vorgeschlagen, auch höhere pH-Werte aufweisen können und zum anderen vor allem auch keine Wirkstoffkombinationen wie zu diesem Zweck (Wundbehandlung) als erforderlich genannt umfasst sind. Es wird angenommen, dass die Zubereitung aufgrund der Auswahl des Wirkstoffes in bestimmten Molmassenbereichen zusammen mit entweder wasser-und/ oder lipidfreien Systemen eine besondere Wirksamkeit auf Schleimhäuten entfaltet, wobei es sich hierbei nicht um die Anwendung bei Chlamydien (d.h. sich intrazellular vermehrenden Bakterien) handelt. Vor allem können auf diese Weise geringe Wirkstoffmengen wie 0,01 bis 0,5 Gew.% schon ausreichend sein.

Nachfolgend werden die Inhaltsstoffe der anmeldungsgemäßen Zubereitungen näher beschrieben. Die Mengenangaben beziehen sich auf Gew.%, bezogen auf die Gesamtzubereitung soweit nicht anders angegeben.

### 1. Wirkstoff

Wesentlicher (Haupt-) Wirkstoff in anmeldungsgemäßen Zubereitungen ist Polyhexanid (Polyhexamethylenbiguanid= PHMB) oder ein Salz hiervon, vor allem das Hydrochlorid gemäß folgender Formel: 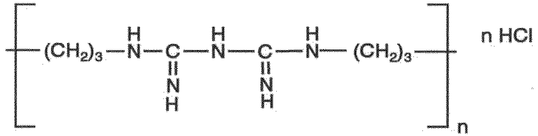 mit n= Anzahl der Einheiten von (C₈H₁₇N₅), molare Masse ca. 183 g/mol, entsprechend der Gesamtmolmasse des eingesetzten Polyhexanids, wie z.B. n=1 bis 140, vorzugsweise 1 bis 85, vor allem 1 bis 30.

Der Wirkstoff liegt frei und nicht liposomal oder in Bakteriophagen vor. Dabei kann die freie Base oder vorzugsweise ein wasserlösliches Salz, z.B. das Hydrochlorid, als Pulver (z.B. gefriergetrocknet) in 100 %iger Konzentration oder in wässriger Lösung eingesetzt werden. Je nach gewünschter galenischer Darreichungsform wird eine flüssige oder feste Form des Polyhexanids eingesetzt.

Bevorzugt wird das Hydrochlorid-Salz eingesetzt.

Die Menge an Polyhexanid- Wirkstoff kann in weiten Bereichen variieren. Üblicherweise enthält das anmeldungsgemäße Mittel den Wirkstoff in Mengen von 0,0001 bis 5 Gew.-%, insbesondere 0,001 bis 2 Gew.-%, vorzugsweise 0,01 bis 1,5 Gew.-%, besonders bevorzugt 0,01 bis 1 Gew.-%, bezogen auf die Gesamtmenge der Zubereitung. Dennoch kann es anwendungsbedingt oder einzelfallabhängig vorteilhaft bzw. erforderlich sein, von den vorgenannten Mengenbereichen abzuweichen, ohne dass der Rahmen der vorliegenden Erfindung verlassen wird.

### 2. Zusatzstoffe

Die anmeldungsgemäßen Zubereitungen weisen weiterhin Zusatzstoffe auf. Diese werden vorzugsweise ausgewählt aus Aromen/Geschmacksstoffen, insbesondere ätherischen Ölen, Vitaminen, sowie galenischen Hilfsstoffen, ausgewählt aus Zuckern, Zuckeraustauschstoffen, Zuckerersatzstoffen, Säuerungsmitteln, Lösungsvermittlern wie z. B. Wasser, Glykol, Glycerin, Verdickern, Süßungsmitteln, Farbstoffen oder Konservierungsmitteln oder Kombinationen hiervon, auch in Abhängigkeit von der galenischen Darreichungsform.

### 2a) Aromen/Geschmacksstoffe

Hierzu gehören vor allem ätherische Öle, die als Aroma- bzw. als Geschmacksstoffe verwendbar sind. Dabei handelt es sich um im Allgemeinen flüchtige Extrakte aus Pflanzen, Pflanzenteilen mit dem dafür charakteristischen Geruch, die vor allem durch Wasserdampfdestillation aus Pflanzen oder Pflanzenteilen gewonnen werden können. Beispielhaft können hier genannt werden: ätherische Öle/Extrakte bzw. Aromastoffe aus Salbei, Nelken, Kamille, Anis, Sternanis, Thymian, Teebaum, Pfefferminze, Minzöl, (Menthol, Cineol), Eukalyptusöl, Mango, Feigen, Lavendelöl, Kamillenblüten, Kiefernnadel, Zypresse, Orangen, Rosenholz, Pflaumen-, Johannisbeer-, Kirscharoma, Birkenblätter, Zimt, Limetten, Grapefruit, Mandarine, Wacholder, Baldrian, Zitronenmelisse, Zitronengras, Palmarosa, Cranberry, Granatapfel, Rosmarin, Ingwer, Ananas, Guave, Echinacea, Efeublätterextrakt, Heidelbeere, Kaki, Melonen u. ä. oder Mischungen hiervon wie Mischungen aus Menthol, Pfefferminz- und Sternanisöl oder Menthol und Kirscharoma.

Diese Aroma- bzw. Geschmacksstoffe können in Mengen von 0,0001 bis 10 Gew.-% (insbesondere als Mischung), vor allem 0,001 bis 6 Gew.-%, bevorzugt 0,001 bis 4 Gew.-%, besonders bevorzugt 0,01 bis 1 Gew.%, bezogen auf die Gesamtzubereitung vorliegen. Anwendungsbedingt oder einzelfallabhängig kann es von Vorteil sein, hiervon abweichende Mengen einzusetzen.

### 2b) Vitamine

Hier kommen sowohl eher wasserlösliche als auch eher fettlösliche Substanzen in Betracht. Je nach Zubereitungsart können feste oder flüssige Substanzen bzw. jeweils Derivate eingesetzt werden. Zu den wasserlöslichen Vertretern zählen insbesondere: Vitamin C bzw. geeignete Derivate hiervon wie - Palmitat, Calcium-Ascorbat / Natrium-Ascorbat, Kalium- Ascorbat, ferner die Vitamine der B- Gruppe wie B1 (Thiamin, z. B. Thiaminnitrat), B2 (Riboflavin), B3 (Niacin, Nicotinsäure und Derivate hiervon wie Niacinamid), B5 (Pantothensäure) wie Calciumpantothenat oder Provitamin B5; B6 (Pyridoxin oder Pyridoxin-Hydrochlorid- oder -phosphat), B7 (Vitamin H, Biotin), B9 (Folsäure), B12 (Cobalamin) oder auch Carotinoide wie Beta / Alpha-Carotin, Beta-Cryptoxanthin, Lutein, Zeaxanthin, Lycopin. Zu den eher fettlöslichen Vitaminen gehören vor allem Vitamin E (Tocopherol und Derivate hiervon wie Tocopherylacetat, Tocopherylpalmitat;Tocopherylsuccinat), ferner Vitamin A (Retinol) und Derivate wie Retinylpalmitat, Retinylacetat, Retinylproprionat; Vitamin D (Cholecalciferol, Ergocalciferol) oder Vitamin K (Phyllochinon).

Besonders bevorzugt werden Vitamin C, insbesondere -palmitat oder Ca-Ascorbat, und/oder Vitamin - E (insbesondere -acetat, succinat,) und/oder Vitamin B5 kombiniert.

Eine weitere geeignete Vitamin- Kombination in anmeldungsgemäßen Zubereitungen umfasst Vitamin A, insbesondere das -Palmitat, Vitamin- E- Acetat, beta-Carotin, Folsäure und /oder Vitamin B5.

Auch können Vitamin C, z. B. Vitamin C- Palmitat zusammen mit Niacinamid, Provitamin B5 oder Pantothensäure kombiniert werden.

Die einzelnen Vitamine bzw. Derivate hiervon werden in den dafür geeigneten Mengen zwischen 0,01 und 3% jeweils, insgesamt z. B. in einer Menge von 0,05 bis 5 Gew.%, bezogen auf die Gesamtgewichtsmenge der Zubereitung, eingesetzt. Vitamine können auch als Farbstoffe eingesetzt werden wie Riboflavin, Carotinoide, andere B-Vitamine oder Mischungen hiervon wie nachfolgend beschrieben.

### 2c) Galenische Hilfsstoffe

### 2c.1) Zucker einschließlich Zuckeraustauschstoffe

Zu den galenischen Hilfsstoffen gehören insbesondere Zucker, einschließlich Zuckeraustauschstoffe, die bei festen Darreichungsformen der anmeldungsgemäßen Zubereitungen als Träger dienen. Vorzugsweise werden damit Lutschbonbons, Lutschtabletten, insbesondere Hartkaramellen hergestellt.

Als Zucker eignen sich Zucker aller Art und/oder Zuckeraustauschstoffe. Im Gegensatz zu den meist intensiv schmeckenden Zuckern werden Zuckeraustauschstoffe technologisch wie Saccharose eingesetzt, d. h. sie besitzen einen physiologischen Brennwert.

Die Süßkraft der Zuckeraustauschstoffe entspricht in weiten Grenzen etwa der von Saccharose. Zuckeraustauschstoffe verfügen im Gegensatz zu Saccharose aber über eine Insulin unabhängige Metabolisierung (vorteilhaft z. B. für Diabetiker). Auch kann hier eine geringere kariogene Wirkung vorliegen. Sie sind daher anmeldungsgemäß bevorzugt.

Beispiele für Zucker sind Saccharose, Glucose, Glucosesirup, Dextrose, Mannose, Fructose, Galactose, Ribose, Arabinose, Xylose, oder Sorbose sowie deren Mischungen untereinander. Zuckeraustauschstoffe sind insbesondere ausgewählt aus Zuckeralkoholen (reduzierte Zucker) wie Glycerin, Threit und Erythrit, Adonit, Arabit, Galactit(ol), Sorbit(ol), Mannit(ol), Xylit, Isomalt, Maltitsirup, Lactit, Leucrose, Fructooligosacchariden, Glucanen und Polyglucose. Besonders bevorzugte Zucker/Zuckeraustauschstoffe sind Glucosesirup, Maltitsirup, Isomalt oder sowie Mischungen hiervon. Besonders bevorzugt werden anmeldungsgemäß Zuckeraustauschstoffe wie beschrieben, vor allem Isomalt, Maltit, Mannit (Mannitol oder Xylit (Xylitol) oder Mischungen hiervon oder Zuckeraustauschstoffe in Kombination mit Zuckern eingesetzt, wobei vorzugsweise Zuckeraustauschstoffe im Überschuss (mehr als 50% der Mischung) vorliegen.

Die Gesamtmenge an Zuckern und/oder Zuckeraustauschstoffen kann in weiten Bereichen variieren. Sie liegt im allgemeinen bei festen Zubereitungen (worin diese Substanzen als Träger dienen) im Bereich von 80 bis 99 Gew.-%, insbesondere 85 bis 99 Gew.-%, bevorzugt 90 bis 98 Gew.-%, besonders bevorzugt 93 bis 98 Gew.-%, bezogen auf die Gesamtmenge des Zubereitung.

Weitere galenische Hilfsstoffe sind:

### 2c.2) Lösungsvermittler

Als Lösungsvermittler eignen sich insbesondere hydrophile Lösungsvermittler wie Wasser, Kochsalzlösung, Glycerin, Polyethylenglykole verschiedener molarer Masse wie 200 bis 400 g/mol. Weiterhin geeignet sind Lösungsvermittler wie nicht ionische Mittel wie z. B. polyoxyethylierte Fettsäuren, polyoxyethylierte Fettalkohole, wie Polysorbate, z. B. Polysorbat 20, Poloxamere (Polyethylenglykol-Polypropylen-Block-Copolymere wie z.B. solche unter dem Handelsnamen Lutrol® erhältliche Produkte mit geeigneter Molmasse).

Alkohole, insbesondere einwertige Alkohole, vor allem Ethanol, oder auch n-Propanol/Isopropanol, oder ggf. Glycerin, sind anmeldungsgemäß nicht erforderlich und bevorzugt nicht vorhanden.

### 2c3) Säuerungsmittel

Säuerungsmittel sind insbesondere Puffer wie Phosphat- oder Citratpuffer sowie Säuren wie Zitronensäure, Weinsäure, Sorbinsäure, vor allem Kaliumcitrat, Natriumcitrat, Puffer wie Natrium(hydrogen)-phosphat/carbonat, Salzsäure u.a.; oder Mischungen hiervon.

### 2c4) Verdicker

Verdicker sind z. B. Arabisches Gummi; Cellulosederivate, z. B. Hydroxyethylcellulose, Hy-droxypropylcellulose, Methylcellulose, Methylhydroxyethylcellulose; Dextrine wie Cyclodextrin, Maltodextrin, Dextrin Guargalactomannan, Dextran; Gelatine, Polydextrose, Chitosan, Chitosanhydrochlorid; Polyethylen-/propylenglycol; Gelatine; Siliciumdioxid, Magnesiumstearat und Xanthan; Zucker wie die vorstehend genannten, Saccharose; Salze wie Natriumchlorid, oder Mischungen aus vorgenannten Substanzen.

### 2c5) Süßungsmittel

Süßungsmittel sind Zuckerersatzstoffe, insbesondere Sucralose; Honig; synthetische Süßstoffe wie Aspartam oder Analoga; Saccharin-Na, oder Mischungen hiervon oder andere, dem Fachmann zu diesem Zweck bekannte Süßungsmittel wie z.B. Acesulfam (beispielsweise auch Acesulfam-K), Cyclamat, Glycyrrhizin, Dulcin, Saccharin (beispielsweise auch Natrium-saccharin und/oder Calciumsaccharin), Rebaudiosid (z. B. Rebaudiosid A), Steviosid, Naringin-Dihydrochalkon, Monellin, Neohesperidin-Dihydrochalkon (NHDC), Sucralose, Thaumatin, Neotam sowie deren Mischungen und Kombinationen, insbesondere Aspartam und/oder Acesulfam, bevorzugt Aspartam und Acesulfam.

Konservierungsmittel können bei Bedarf eingesetzt und dabei ausgewählt werden aus Kaliumsorbat, Methyl-Ethylparaben, Natriumbenzoat und ähnlichen dem Fachmann zu diesem Zweck bekannten Substanzen oder Gemischen hiervon.

### 2c6) Farbstoffe

Geeignete Farbstoffe sind vor allem natürliche wie B-Vitamine, Pflanzenextrakte wie z.B. Rote Beete und in Lebensmitteln und/oder Arzneimitteln zulässige Farbstoffe.

Je nach Wahl bzw. Anteil der galenischen Hilfsstoffe werden feste Darreichungsformen erhalten. Der höhere Anteil an galenischem Hilfsstoff kann dann als Träger bezeichnet werden (s.u.): so kann bei festen Produkten ein oder mehrere Zucker/Zuckeraustauschstoffe als anteilshöchster galenischer Hilfsstoff (=Träger) vorhanden sein.

### Bevorzugte Ausführungsformen

Bevorzugte Ausführungsformen umfassen insbesondere feste, im Wesentlichen wasserfreie (d.h. weniger als 5 Gew%, insbesondere weniger als 2 Gew.% wasserhaltig), vor allem lutschbare, Darreichungsformen. Dabei handelt es sich insbesondere um Lutschbonbons, z. B. Hartkaramellen, oder Pastillen, welche ein Gesamtgewicht von ca. 0,5 bis 5 g aufweisen. Diese sind bevorzugt ungefüllt.

Die festen Darreichungsformen sind vor allem auf Basis von Zuckern und/oder Zuckeraustauschstoffen (Trägerstoffe) wie vorstehend genannt hergestellt, vor allem ausgewählt aus Glucose, Maltose, Isomaltose, Dextrose, Fructose, Xylose, Lactose, Lactulose, Galactose, Inulin, Maltitol, Sorbitol, Xylitol, Mannitol, Erythritol, vorzugsweise Isomalt, Maltitsirup oder Mischungen hiervon, in einer Gesamtmenge bezogen auf die Gesamtzubereitung, von 85 bis 99 Gew.%, vorzugsweise 90 bis 98 Gew.%.

Bevorzugte Darreichungsformen umfassen ungefüllte Hartkaramellen/Pastillen der oben beschriebenen Art.

Die Menge an Trägerstoffen wie oben beschrieben kann in festen Darreichungsformen auch aufgeteilt werden in einen äußeren Hüllungsteil (Mantel), umfassend Zusatzstoffe, und den wesentlichen Anteil an Träger wie 60 bis 90 % der Gesamtträgerstoffmenge, und einen inneren Füllungsteil, umfassend den antiinfektiven Wirkstoff und ggf. Zusatzstoffe sowie den Rest an Trägerstoffen, der dementsprechend den geringeren Anteil aufweist wie 40 bis 10 % der Gesamtträgerstoffmenge. Bevorzugt ist hierbei das Verhältnis von Trägerstoffanteil/Hülle zu Trägerstoffanteil/Fülle 8:1 bis 3:1, vor allem 6:1 bis 4,5:1.

In den festen Zubereitungen, insbesondere Lutschbonbons, vor allem auf Hartkaramellenbasis, sind vor allem 0,01 bis 1 Gew.% Polyhexanid, vorzugsweise 0,1 bis 1 Gew.% Polyhexanid, oder ein ein Salz wie Hydrochlorid hiervon, insbesondere mit einer mittleren Molmasse von 2300 bis 3100 g/mol, sowie 1 bis 4 Gew.% Zusatzstoffe, ausgewählt aus Aromen/Geschmacksstoffen, Farbstoffen, Vitaminen, Süßungsmitteln, Säuerungsmitteln, Verdickern oder Kombinationen einer oder mehrerer solcher Substanzen vorhanden. Als Träger werden hier vor allem Zuckeraustauschstoffe, ggf. Zucker, vor allem Glucosesirup, flüssiges Isomalt, Maltitsirup, Sorbit, Xyllit oder Mischungen hiervon, eingesetzt, bevorzugt in Mengen von 80 bis 99 Gew.%, bezogen auf das Gesamtgewicht der Zubereitung, vorzugsweise 90 bis 98 Gew.%.

Wie erläutert, liegen die festen Zubereitungen vor allem in Form von Lutschpastillen, insbesondere Hartkaramellen vor. Diese umfassen als Inhaltsstoffe (jeweils in Mengen wie oben angegeben) im Wesentlichen Zucker/Zuckeraustauschstoffe, vor allem Glucosesirup, flüssiges Isomalt, Maltitsirup, oder Mischungen hiervon, als Träger sowie die vorgesehene Menge an Polyhexanid-Wirkstoff und ggf. Zusatzstoffe, insbesondere ausgewählt aus Aroma-/Geschmacksstoffen, insbesondere Aromamischungen wie z.B. Menthol, Sternanisöl, Pfefferminzöl, ferner Säuerungsmittel wie Zitronensäure, Süßungsmittel wie Sucralose, Farbstoffe oder Kombinationen hiervon. Die in der Wärme hergestellten Karamellen können zu geeignet geformten Pastillen (rund, elliptisch) ausgeprägt und nach dem Abkühlen konfektioniert werden zu Einzel- oder Mehrfachgebinden.

Eine besonders bevorzugte Ausführungsform fester Darreichungsformen, insbesondere ausfertigt als Hartkaramelle, Pastille (vor allem ungefüllt), umfasst:
94 bis 98 Gewichtsprozent Träger, ausgewählt aus einem oder mehreren Zuckeraustauschstoffen, insbesondere Isomalt, Maltit oder Mischungen hiervon;
0,1 bis 0,7 Gew.% Polyhexanid, insbesondere in Form des Hydrochlorids; vor allem mit einer mittleren molaren Masse von 2300 bis 3100 g/mol;
0,1 bis 4 Gew.% Zusatzstoffe sowie eine geringe Restmenge (weniger als 2 Gew.%, insbesondere weniger als 1,7 Gew.% Wasser).

Die Zusatzstoffe sind vor allem ausgewählt aus Aromen/Geschmacksstoffen (z.B. Menthol, Pfefferminzöl, Sternanisöl und/oder Kirsche), Säuerungsmitteln, insbesondere Zitronensäure, sowie Süßungsmitteln wie vor allem Sucralose, und/oder Farbstoffen.

Der pH-Wert einer 10%igen wässrigen Lösung einer derartigen Darreichungsform liegt bevorzugt zwischen mehr als 3,5 und 7.

In einer weiteren Ausführungsform werden in der oben beschriebenen Zubereitung statt 94-98 Gew. % Zuckeraustauschstoffe (alleine) eine Mischung hiervon mit Zucker oder Zucker alleine, insbesondere Glucosesirup, und die übrigen Bestandteile wie erwähnt eingesetzt.

In einer anderen Ausführungsform liegen die festen Zubereitungen in Form von gefüllten Lutschbonbons vor, mit einem höheren Teil der galenischen Zusatzstoffe in der Hülle, und einem geringeren Teil in der Füllung. Bei Anwendung wird dann der Wirkstoff im Mund-Rachenraum freigesetzt. Derartige gefüllte Lutschbonbons (Pastillen) auf Basis eines Trägerstoffes, ausgewählt aus Zuckeraustauschstoffen, Zuckern oder Kombinationen hiervon können mit einem äußeren Hüllenteil (Mantel) und einem inneren Füllungsteil vorliegen, wobei im äußeren Hüllungsteil (Mantel) der antiinfektive Wirkstoff und Zusatzstoffe, und 60 bis 90 % der Gesamtmenge an Zucker/Zuckeraustausch-Trägerstoff, und der innere Füllungsteil ggf. Zusatzstoffe sowie den Rest an Trägerstoffen, aufweist.

Insbesondere liegt der Träger, vor allem Zucker/Zuckeraustauschstoffe in der Hülle in Mengen von 60 bis 85 Gew.% und in der Füllung in einer Menge von 5 bis 20 Gew.% vor (bezogen auf das Gesamtgewicht der Zubereitung, wobei insgesamt vorzugsweise nicht mehr als 95% Träger vorhanden sind). Der Polyhexanid-Wirkstoff sowie eventuell vorhandene Zusatzstoffe sind wie oben für die feste Darreichungsform beschrieben vorhanden.

In einer weiteren bevorzugten Ausführungsform weisen die anmeldungsgemäßen Zubereitungen den antiinfektiven Wirkstoff Polyhexanid, mit einer mittleren molaren Masse wie beschrieben, oder ein wasserlösliches Salz hiervon, vor allem das Chlorid, in Kombination mit wenigstens einem, vor allem mehreren Aroma/Geschmacksstoffen, vor allem ätherischen Ölen (Extrakten), auf.

In einer anderen bevorzugten Ausführungsform sind anmeldungsgemäße, insbesondere vorstehend beschriebene Zubereitungen im Wesentlichen oder insbesondere frei von einwertigen Alkoholen, vor allem Ethanol. Geringe Mengen (kleiner 1 Gew. %, vor allem geringer 0,1 Gew. %) können aus dem Vorhandensein anderer Zusatzstoffe resultieren und daher vorhanden sein.

### Herstellung

Feste Darreichungsformen, insbesondere Lutschbonbons, werden auf bekannte Weise hergestellt. Dazu werden zunächst die Trägerstoffe, insbesondere Zucker/Zuckeraustauschstoffe, erwärmt auf Temperaturen von z.B. 110°C bis 150°C. Dazu können dann die übrigen Zusatzstoffe (wie Aroma, Zitronensäure und Farbstoffe) sowie der Wirkstoff eindosiert werden. Nach dem Abkühlen auf Temperaturen, bei denen die Zubereitungen noch nicht fest sind, können diese in eine geeignete Form (vorzugsweise rund oder elliptisch) gepresst und sodann vollständig auf Raumtemperatur gekühlt werden.

Pastillen (wie Lutschbonbons), Hartkaramellen können auch hergestellt werden, indem der Träger (z. B. Glucosesirup, flüssiges Isomalt, Maltitsirup, Xyllit, Sorbit) mit der Zubereitungs-Grundmischung (Wirkstoff, Zusatzstoffe) vermengt wird und anschließend in dafür bekannten Vorrichtungen als Strang ausgetragen und in geeignet große Teile geschnitten wird.

Ein besonders geeignetes Verfahren umfasst folgende Herstellungsschritte:
Die Pastillen-Masse wird aus verflüssigtem Isomalt hergestellt. Diesem wird Sucralose hinzugefügt und eingemischt (= Grundrezeptur). Die Grundrezeptur wird gekocht und danach vakuumiert. Über Dosierpumpen werden die Aromamischung (z.B. Menthol, Sternanisöl, Pfefferminzöl), Zitronensäure, ein Farbstoff (z.B. eines der vorgenannten Vitamine B) sowie Polyhexanid hinzugegeben und eingemischt. Die Hartkaramellmasse wird auf dem Stahlband temperiert. Die fertig gekochte und temperierte Pastillen-Masse wird vom Stahlband der Kochanlage in den Kegelroller befördert. Der im Strangformer ausgezogene Pastillenstrang wird in der Prägeanlage zu Pastillen ausgeprägt und anschließend im Kühltunnel abgekühlt.

Bei gefüllten Pastillen mit verschiedenen Hüllungs- bzw. Füllungsteilen wird ein Füllungsteil mit der oben beschriebenen jeweiligen Zubereitungsmischung (z. B. enthaltend flüssiges Isomalt) vermischt und sodann in einer geeigneten Vorrichtung (Doppelrohrführung) der Hüllungsteil (z.B. mit Maltitsirup) eingefüllt. Beide Produktstränge werden dann gleichzeitig ausgetragen und portioniert.

Kautabletten können hergestellt werden, indem man der Basis (z.B. 75 bis 88 Gew. % Dextrose oder Polydextrose) die Zubereitungsmischung in üblichen Apparaturen hinzufügt.

Die festen Zubereitungen werden vereinzelt und/oder in dafür vorgesehene Beutel, Sachets oder Blisterpackungen verpackt. Die Zubereitungen sind lagerstabil.

### Anwendung

Die anmeldungsgemäßen Zubereitungen werden vorzugsweise eingesetzt zur topisch-oralen unterstützenden, protektiven/kurativen Anwendung auf Schleimhäuten, insbesondere bei Menschen, im Mund-Hals-Nasen-und/oder Rachenraum bei Irritationen und Reizungen wie Halsschmerzen, einhergehend mit Halskratzen und/oder Heiserkeit, die durch Bakterien wie z.B. alphahämolysierende Streptokokken, Streptococcus pyogenes, Streptococcus Pneumoniae, Pseudomonas aeruginosa, Staphylococcus aureus, Candida albicans, Enterococcus faecalis, Haemophilus Influenza, Candida albicans, Staphylococcus aureus verursacht sind sowie Entzündungen, die als Folge einer solchen Infektionen hervorgerufen werden. Reizungen durch Chlamydien und derartige Organismen werden mit anmeldungsgemäßen Zubereitungen nicht erfasst. Zubereitungen zur Behandlung der Augen werden erfindungsgemäß bevorzugt nicht umfasst. Insbesondere werden die anmeldungsgemäßen oralen Anwendungsformen, wie vor allem pharmazeutische Zubereitungen, Medizinprodukte (zu denen Kosmetikprodukte ((nicht therapeutisch), Medizinprodukte, und Arzneimittel (therapeutisch) gehören) eingesetzt. Dazu gehören: vor allem lutschbare Pastillen, Hartkaramellen (ungefüllt), gefüllte Pastillen (Bonbons), Lutschtabletten-. Aus den Zubereitungen wird der Wirkstoff durch die Anwendung freigesetzt. Bevorzugt liegen die Zubereitungen in Form von Medizinprodukten, insbesondere in Form von Hartkaramellen vor. Pastillen, Hartkaramellen sind insbesondere Medizinprodukte.

Die anmeldungsgemäßen Mittel können daher insbesondere verwendet werden zur Herstellung eines therapeutischen Mittels, oder auch eines nicht therapeutischen Mittels, bzw. können verwendet werden als Therapeutikum oder Kosmetikum für die Schleimhautapplikation bei Reizzuständen wie Halsschmerzen, die eventuell auch mit Heiserkeit, Halskratzen einhergehen, im Mund-Nasen-Rachenraum, die z.B. durch Bakterien wie alphahämolysierende Streptokokken, Streptococcus pyogenes, Streptococcus pneumoniae, Pseudomonas aeruginosa, Staphylococcus aureus, Candida albicans, Enterococcus faecalis, Haemophilus influenza, Candida albicans, Staphylococcus. aureus verursacht sind sowie bei entzündlichen Zuständen als Folge einer solchen Infektion.

Die anmeldungsgemäß, wie beschrieben hergestellten Mittel sind fest, vor allem lutschbar (Lutschbonbons, Lutschpastillen, lutschbare Hartkaramellen).

### Beispiele

Die Erfindung wird anhand der nachfolgenden Beispiele, die den beschriebenen Gegenstand jedoch nicht beschränken, näher erläutert.

**Beispiel 1 Lutschbonbons zuckerfrei**

| Inhaltsstoff | Chemische Bezeichnung / Funktion | Menge [g] |
|---|---|---|
| Isomalt flüssig Typ ST | Zuckeraustauschstoff E953 | 97,2142 |
| Sucralose | Süßstoff E955 | 0,0808 |
| Menthol | Geschmacksgebung | 0,0265 |
| Sternanisöl | Geschmacksgebung | 0,0208 |
| Pfefferminzöl | Geschmacksgebung | 0,0069 |
| Zitronensäure anhydro | Säuerungsmittel | 0,4269 |
| Farbstoff | Farbgebung | 0,1242 |
| Polyhexanid Lösung* | Wirkstoff | 0,5000 |
| Restfeuchte (Wasser) | | 1,5996 |
| 100,000 | | |

| | | |
|---|---|---|
| • Polyhexanid-Lösung: 20%ige, wässrige Lösung von Poly-Hexamethylene-Biguanide-Hydrochlorid (=PHMB = Polyhexanid), mittlere molare Masse: 2300-3100 g/mol. | | |

| Inhaltsstoff | Chemische Bezeichnung / Funktion | Menge [g] |
|---|---|---|
| Glucosesirup | Zucker | 97,2142 |
| Sucralose | Süßstoff E955 | 0,0808 |
| Menthol | Geschmacksgebung | 0,0265 |
| Kirscharoma | Geschmacksgebung | 0,0277 |
| Zitronensäure anhydro | Säuerungsmittel | 0,4269 |
| Farbstoff | Farbgebung | 0,1242 |
| Polyhexanid Lösung * | Wirkstoff | 0,5000 |
| Restfeuchte (Wasser) | | 1,5996 |
| 100,000 | | |

| | | |
|---|---|---|
| • Polyhexanid Lösung: 20%ige, wässrige Lösung von Poly-Hexamethylene-Biguanide-Hydrochlorid (=PHMB = Polyhexanid), mittlere molare Masse: 2300-3100 g/mol. | | |

**Beispiel 3 Lutschbonbons gefüllt**

| Inhaltsstoff | Chemische Bezeichnung / Funktion | Menge [g] |
|---|---|---|
| Im Mantel enthalten | | |
| Isomalt flüssig Typ ST | Zuckeraustauschstoff E953 | 78,971 |
| Sucralose | Süßstoff E955 | 0,009 |
| Aroma | Geschmacksgebung | 0,639 |
| Zitronensäure anhydro | Säuerungsmittel | 1,078 |
| Polyhexanid Lösung* | Wirkstoff | 0,500 |
| Farbstoff | Farbgebung | 0,163 |
| Restfeuchte (Wasser) | | 1,640 |

| in der Füllung enthalten | | |
|---|---|---|
| Maltitsirup | Zuckeraustauschstoff E963 | 13,690 |
| Zitronensäure anhydro | Säuerungsmittel | 0,226 |
| Aroma | Geschmacksgebung | 0,531 |
| Restfeuchte (Wasser) | | 2,553 |
| | | 100,000 |

| | | |
|---|---|---|
| • Polyhexanid Lösung: 20i%ge, wässrige Lösung von Poly-Hexamethylene-Biguanide-Hydrochlorid (=PHMB = Polyhexanid), mittlere molare Masse: 2300-3100 g/mol. | | |

## Patentansprüche

1. Bakteriophagenfreie Zubereitung zur topisch-oralen Anwendung im nicht gefrorenen Zustand auf Schleimhäuten im Mund- Nasen- und Rachenraum bei Menschen, **dadurch gekennzeichnet, dass** sie Polyhexanid (Polyhexamethylenbiguanid), oder ein wasserlösliches Salz hiervon, als antiinfektiven Wirkstoff, mit einer molaren Masse von 2300 bis 3100 g/mol und einen oder mehrere Zusatzstoffe, ausgewählt aus Aroma-/Geschmacksstoffen, Vitaminen, sowie galenischen Hilfsstoffen, ausgewählt aus Zuckern, Zuckeraustauschstoffen, Zuckerersatzstoffen, Säuerungsmitteln, Lösungsvermittlern, Verdickern, Süßungsmitteln, Farbstoffen, Konservierungsmitteln, oder Kombinationen hiervon aufweist, wobei die Zubereitung im Wesentlichen frei ist von weiteren antiinfektiven Wirkstoffen, und als feste Dareichungsform mit Zuckern und/oder Zuckeraustauschstoffen als Träger vorliegt.

2. Zubereitung nach Anspruch 1, **dadurch gekennzeichnet, dass** sie als pharmazeutische Zubereitung vorliegt.

3. Zubereitung nach Anspruch 1 oder 2, **dadurch gekennzeichnet, dass** sie in Form einer im wesentlichen wasserfreien festen Zubereitung auf Zucker/-Zuckeraustauschstoffbasis als Trägerstoff in Form eines Lutschbonbons vorliegt.

4. Zubereitung nach einem der Ansprüche 1 bis 3 zur topisch-oralen therapeutischen oder nicht therapeutischen protektiven und/oder unterstützenden, oder kurativen Anwendung auf Schleimhäuten im Mund-Nasenraum und/oder Rachen bei infektiösen, nicht durch Chlamydien verursachten und/oder entzündlichen Zuständen.

5. Zubereitung nach einem der Ansprüche 1 bis 4, **dadurch gekennzeichnet, dass** sie in Form eines Medizinproduktes zur topisch-oralen therapeutischen unterstützenden Anwendung im Hals-Rachenraum bei infektiösen, nicht durch Chlamydien verursachten und/oder entzündlichen Zuständen vorliegt.

6. Zubereitung nach einem der Ansprüche 1 bis 5, **dadurch gekennzeichnet, dass** sie Polyhexanid oder ein wasserlösliches Salz hiervon in Kombination mit wenigstens einem Aroma-/Geschmacksstoff aufweist.

7. Zubereitung nach einem der Ansprüche 1 bis 6 , **dadurch gekennzeichnet, dass** sie in Form einer festen lutschbaren Hartkaramelle oder Pastille vorliegt, umfassend 0,01 bis 1 Gew.% Polyhexanid oder ein wasserlösliches Salz hiervon, mit einer mittleren Molmasse von 2300 bis 3100 g/mol, 1 bis 4 Gew.% Zusatzstoffe, ausgewählt aus Aromen/Geschmacksstoffen, Farbstoffen, Vitaminen, Süßungsmitteln, Säuerungsmitteln, Verdickern oder Kombinationen einer oder mehrerer solcher Substanzen, sowie 80 bis 99 Gew.%, bezogen auf das Gesamtgewicht der Zubereitung, Trägerstoffe, ausgewählt aus Zuckeraustauschstoffen, Zuckern oder Mischungen hiervon.

8. Zubereitung nach Anspruch 7, **dadurch gekennzeichnet, dass** sie in Form eines Lutschbonbons auf Basis eines Trägerstoffes, ausgewählt aus Zuckeraustauschstoffen, Zuckern oder Kombinationen hiervon mit einem äußeren Hüllenteil (Mantel) und einem inneren Füllungsteil vorliegt, wobei im äußeren Hüllungsteil (Mantel) der antiinfektive Wirkstoff Polyhexanid oder ein wasserlösliches Salz hiervon, und Zusatzstoffe, und 60 bis 90 % der Gesamtmenge an Zucker/Zuckeraustausch-Trägerstoff, und der innere Füllungsteil ggf. Zusatzstoffe sowie den Rest an Trägerstoffen, aufweist.

9. Zubereitung nach einem der Ansprüche 7 oder 8, **dadurch gekennzeichnet, dass** der oder die Trägerstoffe ausgewählt ist/sind aus Glucosesirup, flüssigem Isomalt, Maltitsirup oder Mischungen hiervon.

10. Zubereitung nach einem der Ansprüche 1 bis 9, **dadurch gekennzeichnet, dass** sie Polyhexanid oder das Hydrochlorid-Salz hiervon in einer Menge von 0,01 bis 1 Gew.%, bezogen auf die Gesamtmenge der Zubereitung, aufweist.

11. Bakteriophagenfreie, feste Zubereitungen gemäß Anspruch 1 zur topischen Anwendung für die Schleimhautapplikation bei Reizzuständen wie Halskratzen und/oder Halsschmerzen, die eventuell mit Heiserkeit einhergehen sowie entzündlichen Zuständen, im Mund-Nasen-Rachenraum, die durch Bakterien wie alphahämolylierende Streptokokken, Streptococcus pyogenes, Streptococcus pneumoniae, Pseudomonas aeruginosa, Staphylococcus aureus, Candida albicans, Enterococcus faecalis, Haemophilus influenza, Candida albicans, Staphylococcus aureus verursacht werden sowie bei entzündlichen Zuständen als Folge dieser Infektionen im Mund-Nasen-Rachenraum.

## Claims

1. Bacteriophage-free preparation for topical-oral application in the non-frozen state on mucous membranes in the mouth, nose and throat of humans, **characterised in that** it comprises polyhexanide (polyhexamethylene biguanide), or a water-soluble salt thereof, as an anti-infective active substance, having a molecular weight of 2300 to 3100 g/mol, and one or more additives selected from flavouring substances/flavours, vitamins, as well as galenic auxiliaries selected from sugars, sugar substitution products, sugar substitutes, acidifiers, solubilisers, thickeners, sweeteners, colourants, preservatives, or combinations thereof, wherein the preparation is essentially free of additional anti-infective active substances and exists as a solid presentation form with sugars and/or sugar substitution products as the carrier.

2. Preparation according to claim 1, **characterised in that** it exists as a pharmaceutical preparation.

3. Preparation according to claim 1 or 2, **characterised in that** it exists in the form of an essentially anhydrous solid preparation on a sugar/sugar substitution product basis as the carrier in the form of a lozenge.

4. Preparation according to one of claims 1 to 3 for the topical - oral therapeutic or non-therapeutic protective and/or supportive or curative application on mucous membranes in the mouth, nose and/or throat for infections not caused by chlamydia and/or inflammatory conditions.

5. Preparation according to one of claims 1 to 4, **characterised in that** it exists in the form of a medicinal product for topical - oral therapeutic supportive application in the throat for infections not caused by chlamydia and/or inflammatory conditions.

6. Preparation according to one of claims 1 to 5, **characterised in that** it comprises polyhexanide or a water-soluble salt thereof in combination with at least one flavouring substance/flavour.

7. Preparation according to one of claims 1 to 6, **characterised in that** it exists in the form of a solid, suckable hard caramel or lozenge, containing 0.01 to 1 wt. % polyhexanide or a water-soluble salt thereof, having an average molecular weight of 2300 to 3100 g/mol, 1 to 4 wt. % additives selected from flavouring substances/flavours, colourants, vitamins, sweeteners, acidifiers, thickeners or combinations of one or more such substances, as well as 80 to 99 wt. %, based on the total weight of the preparation, of carriers selected from sugar substitution products, sugars or mixtures thereof.

8. Preparation according to claim 7, **characterised in that** it exists in the form of a lozenge, based on a carrier, selected from sugar substitution products, sugars or combinations thereof, with an outer shell part (casing) and an inner filling part, wherein the anti-infective active substance in the outer shell part (casing) comprises polyhexanide or a water-soluble salt thereof, and additives, and 60 to 90 % of the total amount of sugar/sugar substitute carrier, and the inner filling part optionally comprises additives as well as the remainder of the carrier materials.

9. Preparation according to one of claims 7 or 8, **characterised in that** the carrier(s) is/are selected from glucose syrup, liquid isomalt, maltitol syrup or mixtures thereof.

10. Preparation according to one of claims 1 to 9, **characterised in that** it comprises polyhexanide or the hydrochloride salt thereof in an amount of 0.01 to 1 wt %, based on the total amount of the preparation.

11. Bacteriophage-free, solid preparation according to claim 1 for topical use for application on mucous membranes in case of irritations such as a raw and/or sore throat which are possibly associated with hoarseness as well as inflammatory conditions in the mouth, nose and throat caused by bacteria such as alpha-haemolysing streptococci, Streptococcus pyogenes, Streptococcus pneumoniae, Pseudomonas aeruginosa, Staphylococcus aureus, Candida albicans, Enterococcus faecalis, Haemophilus influenzae, Candida albicans, Staphylococcus aureus as well as in case of inflammatory conditions as a consequence of these infections in the mouth-nose-throat.

## Revendications

1. Préparation exempte de bactériophages pour l'application topique-orale dans un état non congelé sur les muqueuses dans la cavité buccale, nasale et de la gorge chez l'homme, **caractérisée en ce qu'**elle contient du polyhexanide (polyhexaméthylène biguanide), ou un sel soluble dans l'eau de celui-ci, comme principe actif anti-infectieux, présentant une masse molaire de 2300 à 3100 g/mole, et un ou plusieurs additifs, choisis parmi les arômes/substances gustatives, les vitamines ainsi que les adjuvants galéniques, choisis parmi les sucres, les produits de substitution du sucre, les succédanés de sucre, les acidifiants, les promoteurs de solubilisation, les épaississants, les édulcorants, les colorants, les conservateurs ou les combinaisons de ceux-ci, la préparation étant essentiellement exempte d'autres principes actifs anti-infectieux et se trouvant sous une forme posologique solide avec des sucres et/ou des produits de substitution du sucre comme véhicule.

2. Préparation selon la revendication 1, **caractérisée en ce qu'**elle se trouve sous forme de préparation pharmaceutique.

3. Préparation selon la revendication 1 ou 2, **caractérisée en ce qu'**elle se trouve sous forme d'une préparation solide essentiellement anhydre à base de sucre/produit de substitution de sucre comme excipient sous forme d'un bonbon à sucer.

4. Préparation selon l'une quelconque des revendications 1 à 3 pour une utilisation topique-orale, thérapeutique ou non thérapeutique, protectrice et/ou de soutien ou curative sur les muqueuses dans l'espace buccal-nasal et/ou dans la gorge dans le cas d'états infectieux, non provoqués par des chlamydias, et/ou inflammatoires.

5. Préparation selon l'une quelconque des revendications 1 à 4, **caractérisée en ce qu'**elle se trouve sous forme d'un produit médical pour une utilisation topique-orale, thérapeutique de soutien dans la cavité de la gorge dans le cas d'états infectieux, non provoqués par des chlamydias, et/ou inflammatoires.

6. Préparation selon l'une quelconque des revendications 1 à 5, **caractérisée en ce qu'**elle contient du polyhexanide ou un sel soluble de celui-ci en combinaison avec au moins un(e) arôme/substance gustative.

7. Préparation selon l'une quelconque des revendications 1 à 6, **caractérisée en ce qu'**elle se trouve sous forme d'un caramel dur ou d'une pastille à sucer, comprenant 0,01 à 1% en poids de polyhexanide ou d'un sel soluble dans l'eau de celui-ci, présentant une masse molaire moyenne de 2300 à 3100 g/mole, 1 à 4% en poids d'additifs, choisis parmi les arômes/substances gustatives, les colorants, les vitamines, les édulcorants, les acidifiants, les épaississants ou les combinaisons d'une ou de plusieurs de ces substances, ainsi que 80 à 99% en poids, par rapport au poids total de la préparation, d'excipients, choisis parmi les produits de substitution du sucre, les sucres ou leurs mélanges.

8. Préparation selon la revendication 7, **caractérisée en ce qu'**elle se trouve sous forme d'un bonbon à sucer à base d'un excipient, choisi parmi les produits de substitution du sucre, les sucres ou leurs combinaisons, présentant une partie d'enrobage externe (enveloppe) et une partie de remplissage interne, la partie d'enrobage (enveloppe) présentant le principe actif anti-infectieux polyhexanide ou un sel soluble dans l'eau de celui-ci et des additifs et 60 à 90% de la quantité totale d'excipient de type sucre/produit de substitution de sucre et la partie de remplissage interne présentant le cas échéant des additifs ainsi que le reste des excipients.

9. Préparation selon l'une quelconque des revendications 7 ou 8, **caractérisée en ce que** ledit ou lesdits excipients est/sont choisi(s) parmi le sirop de glucose, l'isomalt liquide, le sirop de maltitol ou leurs mélanges.

10. Préparation selon l'une quelconque des revendications 1 à 9, **caractérisée en ce qu'**elle présente le polyhexanide ou le sel de chlorhydrate de celui-ci en une quantité de 0,01 à 1% en poids, par rapport à la quantité totale de la préparation.

11. Préparations solides, exemptes de bactériophages selon la revendication 1 destinées à une utilisation topique pour une application sur les muqueuses dans le cas d'états d'irritation, comme les chatouillements et/ou les douleurs de la gorge, qui vont éventuellement de pair avec un enrouement, ainsi que dans le cas d'états inflammatoires, dans la cavité buccale-nasale et de la gorge, qui sont provoqués par des bactéries telles que le streptocoque alpha-hémolytique, Streptococcus pyogenes, Streptococcus pneumoniae, Pseudomonas aeruginosa, Staphylococcus aureus, Candida albicans, Enterococcus faecalis, Haemophilus influenza, Candida albicans, Staphylococcus aureus, ainsi que dans le cas d'états inflammatoires suite à ces infections dans la cavité buccale-nasale et de la gorge.
